# EUROPEAN PATENT APPLICATION

(11) **EP 4 574 986 A2**
(43) Date of publication of application: **25.06.2025**
(21) Application number: 24218993.4
(22) Date of filing: 11.12.2024
(51) Int. Cl.: C12P 13/02, C07C 231/06, C07C 233/09, C08F 120/56

(54) **HEAD SPACE / GAS CHROMATOGRAPHY FOR REACTION MONITORING OF ACRYLAMIDE SYNTHESIS**

(30) Priority: 18.12.2023 CN 202311752347
(71) Applicant: SNF Group, 42160 Andrézieux-Bouthéon (FR)
(72) Inventor: GUILLARD, Alexis, 42160 Andrézieux-Bouthéon (FR); KIEFFER, Johann, 42160 Andrézieux-Bouthéon (FR); LEGRAS, Benoît, 42160 Andrézieux-Bouthéon (FR); CHARVOLIN, Annabelle, 42160 Andrézieux-Bouthéon (FR); FAVERO, Cédrick, 42160 Andrézieux-Bouthéon (FR); CORPELET, Antoine, 42160 Andrézieux-Bouthéon (FR); LING, Jing, Taixing City, 225400 (CN); BOISSE, Nicolas, Taixing City, 225400 (CN)
(74) Representative: Ipsilon Lyon

(57) **Abstract**

The invention relates to a process for producing an aqueous acrylamide solution, comprising:
(a) combining water and at least one biocatalyst having nitrile hydratase activity to provide a slurry;
(b) feeding acrylonitrile into a reactor comprising said slurry to provide a reaction mixture; and
(c) monitoring said reaction mixture by online GC to measure a concentration of acrylonitrile in reactor's headspace with several detection technologies selected in the group consisting of Flame Ionization Detector, Mass Spectrometry, Thermal Conductivity Detector, Electron Capture Detector, Nitrogen-Phosphorus detector and vacuum ultraviolet detector.

## Description

### FIELD OF THE ART

The present disclosure generally relates to the field of acrylamide synthesis, and more particularly, to reaction monitoring of acrylamide synthesis by gas chromatography (GC) on reactor head space., called online-GC. As such, the present disclosure relates to a process for producing aqueous acrylamide solution by hydrolyzing acrylonitrile in an aqueous solution in the presence of a biocatalyst, wherein the method comprises in-line monitoring of the acrylamide synthesis reaction by online-GC. The present disclosure also generally relates to aqueous acrylamide solutions obtainable by said process and use thereof for the synthesis of polyacrylamide.

### BACKGROUND

Acrylamide (AM) has been on the market since the mid-1950s, and the acrylamide market has grown steadily since that time. Acrylamide is used primarily in the production of polyacrylamide, which is used in many application fields including water treatment, oil and gas recovery, papermaking industry, and mining processes. Acrylamide is produced from acrylonitrile (ACN) by hydrolysis reaction in the presence of a catalyst.

Conventionally, production of acrylamide was based on a chemical catalyst process, namely, sulfuric acid-catalyzed hydration reaction or copper-catalyzed hydration which slowly replaced the sulfuric acid process. Enzyme catalyzed acrylamide production processes were developed in the 1980s. Advantages in contrast to the conventional process are low reaction temperature, atmospheric operating pressure, complete conversion, low by-product selectivity and easy downstream processing.

There are two types of nitrile-degradation metabolic pathways existing in microorganisms: nitrilase and nitrile hydratase (NHase) pathways. Three different enzymes are involved in these reactions: nitrilase, (NHase), and amidase.

Nitrilase catalyzes the hydrolysis reaction of nitriles directly into corresponding carboxylic acid and ammonia products. In NHase pathway. NHase hydrolyzes nitriles into the corresponding amide product at first. In the presence of amidase, amides may be further converted into corresponding acid and ammonia products. Methods for producing acrylamide from acrylonitrile in the presence of a biocatalyst, e.g. nitrile hydratase are described in numerous patent publications. The monitoring of such reactions, *i.e.,* in order to measure the concentrations of reaction components, including acrylonitrile and acrylamide, as well as byproducts (e.g. acrylic acid), in such processes, e.g., using HPLC-based detection methods is also known.

Residual acrylonitrile content should be as low as possible after bioconversion of acrylonitrile into acrylamide. Residual acrylonitrile is dependent on the acrylonitrile quality (impurities profile) and of enzyme concentration for bioconversion. Off-line monitoring of acrylonitrile could induce over dosage of enzyme: indeed, the operator will prioritize the conformity, safety and environmental specifications of the production unit; too low enzyme concentration can lead to higher concentration on acrylonitrile in the production reactors causing flammable risk; or in the finishing product and environmental issue ; consequently, acrylamide aqueous solution is generally produced with an overdosage of enzyme with impact on the long-term acrylamide stability (auto-polymerization risk) and global quality with problems of turbidity, color modification, by-product generation etc...) leading to low quality grade of acrylamide for polymerization. The enzyme dosage must be continuously adjusted for an optimum and cheaper acrylamide quality, without an additional posttreatment required.

It is an object of the present disclosure to provide an improved process for producing an aqueous acrylamide solution whereby the reaction status is monitored using gas chromatography (GC). On-line Gas Chromatography (online-GC) is an analytical technique which does not require specific sample preparation in the case of gas head space reactor analysis. This technology also reduces the use of consumables and spare parts such as organic solvents, chromatographic columns, or reagents. GC also provides real-time analysis, at a higher frequency compared to conventional technology. On-line GC system is also more sensitive, accurate and reproducible compared to other in-line or on-line technologies as Fourier-Transform Infrared Spectroscopy (FTNIR), Mid-infrared spectroscopy (MIR), High Performance Liquid Chromatography (HPLC) or RAMAN spectroscopy. Analysis period by GC is higher compared to said technologies but perfectly relevant with acrylamide synthesis kinetic. Accordingly, on-line GC monitoring provides sensitivity and robustness and increased manufacturing capacity compared to conventional off-line laboratory techniques such as HPLC or GC and compared to in-line technologies such MIR, RAMAN or FTNIR.

GC technology is a chromatographic technology which separates chemicals accordingly to their volatility and chemistry. The GC detector measures the concentration of analytes. As example, FID detector is based on the principle of ions liberated in the combustion of chemicals while MS is an analytical technique which separates ionized particles according to their charges and to their respective masses (m/z). As such, GC is more suitable for measuring analytes either in aqueous or organic solution and in gas compared to FTNIR, MIR or RAMAN technologies as the power of separation is higher in GC with a lower sensitivity compared to said spectroscopic technologies. Further, GC can identify unknown compounds when mass spectrometry is used, and limits interference phenomenon compared to spectroscopic technologies.

Online-GC technology enables an installation of analyzer around 20 m and more from reactor, without a hypothetical loss of information compared to other technologies as HPLC with a non-representative sample coming to the analyzer or spectroscopic technologies (FTNIR as example) which are using long fiber-optic cables which can generate a lot of information through cutoff phenomenon for example. By using on-line GC technology in such environment, there is no need to put electrical components or Ex-proof equipment. In addition, this installation enables reactor interconnection to generate data over the whole synthesis process.

Monitoring reactor's headspace is easier and demonstrates higher efficiency compared to the analysis of reactor's liquid by Headspace-GC/FID technology due to the limitations from sample's matrix. Those limitations are linked to physical properties of the sample (presence of insoluble material such as catalyst) and represent polymerization risk which cannot be disregarded. Such phenomena are hard to limit by classical Headspace-GC (offline measurement) or by using spectroscopic technologies such FTNIR, RAMAN or MIR. Therefore, this analysis can induce a bias during the analysis and troubleshooting's might also lower the capacity (frequency, accuracy, availability) of these technologies, implying a low sampling frequency and a potential increase in enzyme consumption Thus, online-GC in reactor headspace increases the sensitivity compared to stated technologies, which allow to operate process with higher efficiency. Using GC technology also implies a separation of the compound before detection; therefore, it decreases the risk of interferences, which could not be totally discarded by using spectroscopic technologies. Despite monitoring acrylonitrile, other volatile compounds can also be analyzed as benzene, acrolein, methyl vinyl ketone, oxazole, propionitrile and any other volatile molecules which can be found in acrylonitrile raw material. To allow an accurate quantification of acrylonitrile in gas, a permeation tube of acrylonitrile is installed on the system which allows daily verification of mathematical model accuracy. Coupling this verification to GC technology is ensuring an accurate quantification with a low uncertainty compared to other technologies. Spectroscopic technologies use mathematical models (sophisticated chemometric algorithms) always based on reference techniques (conventional chromatography either liquid or gaseous). So, this reference to a different technique implies to have higher uncertainty. This is not the case when using on-line GC which ensures better accuracy.

Therefore, the full principle of GC technology by implying the analysis of reactor's headspace, by performing chemical separation prior to the detection and by using internal calibration curve allowed to decrease the risk to generate biased results compared to other technologies as spectroscopic technologies such as FTNIR, RAMAN or MIR as spectral interferences which can generate important and not predictable bias.

### BRIEF SUMMARY

The present disclosure generally relates to an improved process for producing an aqueous acrylamide solution. The process comprises a) combining water and at least one biocatalyst having nitrile hydratase activity to provide a slurry; b) feeding acrylonitrile into a reactor comprising said slurry to provide a reaction mixture; and c) monitoring said reaction mixture by on-line GC to measure a concentration of acrylonitrile in the reactor's headspace.

In some embodiments, one or more of (i) the acrylonitrile feed rate and/or (ii) the amount of water and/or (iii) the at least one biocatalyst and/or the amount thereof and/or (iv) the temperature and/or the pH may be adjusted during the reaction process based on the detected concentration of acrylonitrile in the reactor's headspace.

In some embodiments, the sampling loop compressor is not a temperature-controlled loop connected thereto to GC online system.

A "sampling loop compressor" is a component used in gas chromatography (GC) systems to improve the accuracy and efficiency of sampling. It is often integrated into gas monitoring systems, such as those used to detect specific compounds in air or gas samples. For example, in a chromatography system for greenhouse gas monitoring, a "sampling loop compressor" can be used to compress and inject gas samples into the chromatography system, allowing for precise analysis of different gas levels.

In some embodiments, the acrylamide concentration is determined by measuring an online refractive index to optimize mathematical model used by online GC.

In some embodiments, the monitoring of step c) by online GC to measure a concentration of acrylonitrile in the reactor's headspace may be carried out with several detection techniques selected in the group consisting of FID/MS/TCD/NPD/ECD/VUV.

In some embodiments, an online GC system with appropriate detector (FID or MS or ECD or NPD or TCD or VUV) may be positioned close to the reactor with a short gas sampling line. In some embodiments, an online GC system may be placed outside the reactor and connected to head space reactor with gas sampling line. In some specific embodiments, the reactor may comprise a thermostatically controlled sampling line connected to an online GC. In some embodiments, an online-GC system may be positioned in the process and connected to several reactors head space.

In some embodiments, the concentration of acrylonitrile may be within a range of 0 to 15000 ppmv (part per million per volume) and may be measured by online-GC in the reactor's headspace with an accuracy of at most ± 10 % of nominal values more specifically at most ± 5 % of nominal value.

In some embodiments, the concentration of acrylonitrile may be within a range of 0 to 15000 ppmv and may be measured by online-GC in the reactor's headspace with an accuracy of at least ± 100 ppmv, more specifically at least ± 50 ppmv.

In some embodiments, the concentration of acrylonitrile may be within a range of 0 to 5000 ppmv and may be measured by online GC in the reactor's headspace with an accuracy of at most ± 5 % of nominal value, more specifically at most ± 2.5 % of nominal value,

In some embodiments, the concentration of acrylonitrile may be within a range of 0 to 5000 ppmv and may be measured by online-GC in the reactor's headspace with an accuracy of at least ± 50 ppmv, more specifically at least ± 10 ppmv.

In some embodiments, the concentration of acrylonitrile may be within a range of 0 to 300 ppmv and may be measured by online-GC with an accuracy of at most ± 2.5% of nominal value.

In some embodiments, the concentration of acrylonitrile may be within a range of 0 to 300 ppmv and may be measured by online-GC in the reactor's headspace with an accuracy of at least ± 10 ppmv.

In some embodiments, the acrylonitrile feed rate may be adjusted during the process, thereby controlling acrylonitrile accumulation in the reactor.

In some embodiments, the reactor may be a semi-batch reactor, micro reactor or a continuous reactor, continuous reactors in series, or stirred tank reactors in series.

In some embodiments, the reactor can be an arrangement of one or several microreactors operate in fed-batch or continuous.

In some embodiments, the reactor can be an arrangement of one or several microreactors that operate in fed-batch or continuous and classical stirred tank reactors.

In some embodiments, the biocatalyst may comprise 0.0001 to 2 kg dry cells/m³ of the reaction mixture.

In some embodiments, the biocatalyst may be a microbe selected from the group consisting of *Rhodococcus, Aspergillus, Acidovorax, Agrobacterium, Bacillus, Bradyrhizobium, Burkholderia, Escherichia, Geobacillus, Klebsiella, Mesorhizobium, Moraxella, Pantoea, Pseudomonas, Rhizobium, Rhodopseudomonas, Serratia, Amycolatopsis, Arthrobacter, Brevibacterium, Corynebacterium, Microbacterium, Micrococcus, Nocardia, Pseudonocardia, Trichoderma, Myrothecium, Aureobasidium, Candida, Cryptococcus, Debaryomyces, Geotrichum, Hanseniaspora, Kluyveromyces, Pichia, Rhodotorula, Comomonas,* and *Pyrococcus* or may comprise a combination of at least two of any of the foregoing microbes, more specifically the biocatalyst may be selected from the group consisting of *Rhodococcus, Pseudomonas, Escherichia* and *Geobacillus* or a combination of at least two of any of the foregoing.

In some embodiments, the biocatalyst may comprise 0.0001 to 2 kg dry cells/m³ of the reaction mixture and may be a microbe selected from the group consisting of *Rhodococcus, Aspergillus, Acidovorax, Agrobacterium, Bacillus, Bradyrhizobium, Burkholderia, Escherichia, Geobacillus, Klebsiella, Mesorhizobium, Moraxella, Pantoea, Pseudomonas, Rhizobium, Rhodopseudomonas, Serratia, Amycolatopsis, Arthrobacter, Brevibacterium, Corynebacterium, Microbacterium, Micrococcus, Nocardia, Pseudonocardia, Trichoderma, Myrothecium, Aureobasidium, Candida, Cryptococcus, Debaryomyces, Geotrichum, Hanseniaspora, Kluyveromyces, Pichia, Rhodotorula, Comomonas,* and *Pyrococcus* or may comprise a combination of at least two of any of the foregoing microbes, more specifically the biocatalyst may be selected from the group consisting of *Rhodococcus, Pseudomonas, Escherichia* and *Geobacillus* or may alternatively or in addition comprise a nitrile hydratase derived from any of the foregoing microbes or a combination of at least two of any of the foregoing.

In some embodiments, the biocatalyst may be *Rhodococcus rhodochrous* or *Rhodococcus aetherivorans* or a nitrile hydratase derived therefrom.

In some embodiments, said process may further comprise measuring and adjusting temperature of the reaction mixture.

In some embodiments, said process may further comprise maintaining the temperature of the reaction mixture within a range of 10 °C to 35 °C; preferably between 15 and 30°C and more preferably between 20 and 25°C optionally so that the final concentration of acrylonitrile is at most 1000 ppmv.

In some embodiments, the final concentration of acrylonitrile as measured by GC in reactor's headspace is at most 1000 ppmv, at most 500 ppmv, at most 150 ppmv, or more specifically at most 50 ppmv.

In some embodiments, said process may further comprise cooling the reaction mixture.

In some embodiments, said process may further comprise cooling the reaction mixture when the acrylamide concentration reaches 28 wt% to 30 wt%.

In some embodiments, said process may further comprise cooling the reaction mixture until the acrylamide concentration reaches 40 wt% to 50 wt%.

In some embodiments, said process may further comprise maintaining the temperature of the reaction mixture for 10 s to 29 min.

In some embodiments, said process may further comprise maintaining the temperature of the reaction mixture for 30 min to 120 min.

In some embodiments, said process may further comprise maintaining the temperature of the reaction mixture for 121 min to 48 h.

In some embodiments, said process may further comprise cooling the reaction mixture so that temperature of the reaction mixture is within a range of 5 °C to 20°C, more specifically 10 °C to 15 °C. The present disclosure also generally relates to an aqueous acrylamide solution obtainable by a process disclosed herein.

In some embodiments, said aqueous acrylamide solution may be characterized in that: the concentration of the acrylamide solution may be from 15 wt% to 55 wt%; concentration of residual acrylonitrile in the acrylamide solution may be equal or less than 1000 ppm, measured by online-GC and after correlation between acrylonitrile concentration in reactor headspace (ppmv) and concentration in liquid (ppm); and turbidity of the acrylamide solution may be equal or less than 20 NTU measured from filtrated 0.45 µm acrylamide sample.

In some embodiments, the concentration of acrylonitrile in acrylamide head space reactors measured by online-GC may be in the range from 0 to 7500 ppmv and may be measured with an accuracy of at most ± 5 % of nominal value, more specifically at most ± 2.5 % of nominal value.

In some embodiments, the color of said acrylamide solution may be equal or less than 20 Hazen measured with spectrophotometer PtCo (455 nm, cell path length: 10 mm, 25°C) from an acrylamide sample filtrated through a 0.45 µm filter.

In some embodiments, the concentration of said acrylamide solution may be from 34 wt% to 55 wt%, more specifically from 38 wt% to 40 wt%.

In some embodiments, the concentration of said acrylamide solution may be from 35 wt% to 55 wt%, more specifically from 38 wt% to 40 wt%.

In some embodiments, the concentration of said acrylamide solution may be from 38 wt% to 55 wt%.

In some embodiments, the concentration of the residual acrylonitrile in said acrylamide solution measured by online-GC may be equal or less than 150 ppmv, more specifically equal or less than 90 ppmv, more specifically equal or less than 50 ppmv, still more specifically equal or less than 10 ppmv.

In some embodiments, the turbidity of said acrylamide solution may be equal or less than 15 NTU with turbidimeter technology.

The present disclosure also generally relates to use of said aqueous acrylamide solution obtainable by a process disclosed herein in manufacturing of polyacrylamide.

The present disclosure also generally relates to an aqueous acrylamide solution wherein
- the color of the solution is equal or less than 20 Hazen measured with spectrophotometer PtCo (455 nm, cell path length: 10 mm, 25°C) from an acrylamide sample filtrated through a 0.45 µm filter;
- the concentration of acrylamide solution is from 35 to 55 weight %, more specifically from 38 to 40 weight %;
- the turbidity of the solution is equal or less than 15 NTU.

### DETAILED DESCRIPTION

### 1. Overview

According to a first aspect of the present disclosure, there is provided a process for producing an aqueous acrylamide solution. More particularly, there is provided a process for producing an aqueous acrylamide solution comprising combining water and a biocatalyst having nitrile hydratase activity to provide a slurry; feeding acrylonitrile into a reactor comprising said slurry to provide a reaction mixture; and monitoring said reaction mixture head space by on-line GC to measure a concentration of acrylonitrile in gas. In some embodiments, an online-GC may be positioned on the reactor. In some embodiments, the reactor may comprise a cooling loop connected thereto, and an online-GC connected in the cooling loop. In some embodiments, the concentration of acrylonitrile may be measured with an accuracy of at least ± 100 ppmv more specifically at least ± 30 ppmv.

According to a second aspect of the present disclosure, there is provided an aqueous acrylamide solution obtainable by said process.. There is provided an aqueous acrylamide solution characterization in that a concentration of acrylonitrile in define head space may be measured by online-GC and correlation with acrylonitrile in aqueous acrylamide is made based on mathematical model which include all variables from process. The concentration of acrylonitrile in head space may be measured with an accuracy of at least ± 50 ppmv, more specifically at least ± 10 ppmv.

In a third aspect of the present disclosure, there is provided use of the aqueous acrylamide solution produced by the disclosed process in manufacturing of polyacrylamide.

As used herein, "GC" refer to Gas Chromatography, "FID" refer to Flame Ionization Detector, "MS" refer to Mass Spectrometry", "TCD" refer to "Thermal Conductivity Detector", "ECD" refer to "Electron Capture Detector", "NPD" refer to Nitrogen-Phosphorus detector", "VUV" refer to Vacuum ultraviolet detector.

As used herein, "biocatalyst" refers to any biocatalyst having at least nitrile hydratase (NHase) activity. The biocatalyst capable of converting acrylonitrile to acrylamide may be a microbe which encodes an enzyme having nitrile hydratase activity (e.g, an NHase) or any part of said microorganism having nitrile hydratase activity. With this regard, it is not relevant whether the microorganism or microbe is naturally encoding nitrile hydratase, or whether it has been genetically modified to encode said enzyme, or whether a microorganism or microbe naturally encoding nitrile hydratase has been modified such as to be able to produce more and/or enhanced nitrile hydratase. Further, it is not relevant whether the enzyme having nitrile hydratase activity is a naturally occurring enzyme or a modified enzyme. The biocatalyst may be selected from said microorganism or microbe, lysed cells of said microorganism, a cell lysate of said microorganism or microbe, or any combination of these. In a very specific embodiment, the biocatalyst is a nitrile hydratase (NHase).

As used herein "Platinum-Cobalt", "PtCo" or Pt/Co refers to a color scale that was first introduced in 1892 by chemist Allen Hazen (1869-1930) to evaluate pollution levels in wastewater. It has since expanded to a common method of comparison of the intensity of yellow-tinted samples. It is specific to the color yellow and is based on dilutions of a 500 ppm platinum cobalt solution. The color produced by one milligram of platinum cobalt dissolved in one liter of water is fixed as one unit of color in platinum-cobalt scale. The ASTM has detailed description and procedures in ASTM Designation D1209, "Standard Test Method for Color of Clear Liquids (Platinum-Cobalt Scale)". Color is measured by visual comparison of the sample with platinum-cobalt standards. One unit of color that produced by 1 mg/L platinum in the form of the chloroplatinate ion. Since very slight amounts of turbidity may interfere with the determination, samples showing visible turbidity are generally clarified by centrifugation. Also, the method is pH dependent.

### 2. Reaction Monitoring by online-GC

The inventors have surprisingly found that the acrylamide synthesis reaction may be monitored by online Gas Chromatography (GC) coupling to specific detectors as at least FID, MS, NPD, TCD, VUV, ECD to achieve an accuracy for the measurement of acrylonitrile concentration in the reaction mixture head space of at least ± 5 ppmv. This accuracy is an order of magnitude lower than what has been possible by conventional offline GC. Preferred detector is FID.

The reactor may be any suitable reactor, such as a semi-batch reactor, a continuous reactor, continuous reactors in series, or stirred tank reactors in series, in some exemplary embodiments, a semi-batch reactor. In some embodiments, an online-GC system may be positioned on each reactor with a short sampling line. In some embodiments, the reactors comprise a cooling loop attached thereto, and an online-GC positioned away than reactors and connected through the gas sampling line. It is contemplated herein that positioning the online-GC away from reactor, with a specific gas sampling line, allowed a better monitoring of each reactor compared to a use of conventional offline GC system with liquid sampling. It is further contemplated that this would result in still further improved precision in the reaction component concentration measurements, e.g. in determining the concentration of acrylonitrile on-line.

In some embodiments, the GC online system is positioned close to the reactors.

### 3. Biocatalyst

The biocatalyst may be fresh (*i.e.,* straight from fermentation); stored, such as stored as frozen (frozen as wet); or dry, before the production of the slurry.

After the fermentation, the biocatalyst slurry may often typically be washed, or otherwise or in addition to suitably treated before entering the slurry or before storage, e.g. by freezing.

The biocatalyst may be any biocatalyst having nitrile hydratase (NHase) activity known in the art.

In accordance with any one of the embodiments of the present disclosure, the biocatalyst capable of converting acrylonitrile to acrylamide may be a microorganism or a microbe which encodes an enzyme having nitrile hydratase activity (e.g, an NHase) or any part of said microorganism or a microbe having nitrile hydratase activity. With this regard, it is not relevant whether the microorganism or a microbe is naturally encoding nitrile hydratase, or whether it has been genetically modified to encode said enzyme, or whether a microorganism or a microbe naturally encoding nitrile hydratase has been modified such as to be able to produce more and/or enhanced nitrile hydratase. Further, it is not relevant whether the enzyme having nitrile hydratase activity is a naturally occurring enzyme or a modified enzyme. The biocatalyst may be selected from said microorganism or a microbe, lysed cells of said microorganism or a microbe, a cell lysate of said microorganism or a microbe, or any combination of these. In a very specific embodiment, the biocatalyst is a nitrile hydratase (NHase).

Microbes encoding nitrile hydratase (e.g. naturally encoding or genetically modified to encode nitrile hydratase) or any part of said microorganism, which can be used as biocatalyst in any one of the embodiments described herein, comprise species belonging to a genus selected from the group consisting of *Rhodococcus, Aspergillus, Acidovorax, Agrobacterium, Bacillus, Bradyrhizobium, Burkholderia, Escherichia, Geobacillus, Klebsiella, Mesorhizobium, Moraxella, Pantoea, Pseudomonas, Rhizobium, Rhodopseudomonas, Serratia, Amycolatopsis, Arthrobacter, Brevibacterium, Corynebacterium, Microbacterium, Micrococcus, Nocardia, Pseudonocardia, Trichoderma, Myrothecium, Aureobasidium, Candida, Cryptococcus, Debaryomyces, Geotrichum, Hanseniaspora, Kluyveromyces, Pichia, Rhodotorula, Comomonas,* and *Pyrococcus.* In exemplary embodiments, the biocatalyst is selected from bacteria of the genus *Rhodococcus, Pseudomonas, Escherichia,* and *Geobacillus.* Typically, the biocatalyst is selected from the group consi ting of *Rhodococcus, Aspergillus, Acidovorax, Agrobacterium, Bacillus, Bradyrhizobium, Burkholderia, Escherichia, Geobacillus, Klebsiella, Mesorhizobium, Moraxella, Pantoea, Pseudomonas, Rhizobium, Rhodopseudomonas, Serratia, Amycolatopsis, Arthrobacter, Brevibacterium, Corynebacterium, Microbacterium, Micrococcus, Nocardia, Pseudonocardia, Trichoderma, Myrothecium, Aureobasidium, Candida, Cryptococcus, Debaryomyces, Geotrichum, Hanseniaspora, Kluyveromyces, Pichia, Rhodotorula, Comomonas,* and *Pyrococcus,* or any part of said microorganism or microbe having nitrile hydratase activity.

In some embodiments the biocatalyst is selected from the group consisting of *Rhodococcus,* e.g. *Rhodococcus pyridinovorans* or *Rhodococcus rhodochrous* or *Rhodococcus aetherivorans, Pseudomonas, Escherichia,* and *Geobacillus,* or any part of said microorganism or microbe having nitrile hydratase activity.

In exemplary embodiments, the biocatalyst is *Rhodococcus aetherivorans* or *Rhodococcus rhodochrous,* or any part of said microorganism or microbe having nitrile hydratase activity. Preferred wild type nitrile hydratase are Rhodococcus rhodochrous J1-H, Rhodococcus rhodochrous M8, Rhodococcus ruber TH, Rhodococcus pyridinovorans MW3, and P.thermophila JCM3095.

Improved (mutant) nitrile hydratases are generally formed by substituting amino acids of a wild-type nitrile hydratase in alpha and/or in beta sub-unit. Amino-acid sequences of wild-type nitrile hydratases to be substituted are made in public in NCBI databases such as GenBank (http://www.ncbi.nlm.nih.gov/) and the like.

The improved nitrile hydratase is characterized by the modification of the amino-acid sequence of a wild type nitrile hydratase, said modification comprising at least one amino-acid modification, preferably at least two amino-acid modifications, more preferably at least three amino-acid modifications on the beta sub-unit chosen from the following list : 14S, 14Q, 14D, 17G, 17E, 17A, 17V, 17W, 43H, 43Q, 43N, 46H, 46T, 48D, 48W, 48D, 57V, 57M, 57G, 69F, 69T, 77P, 77E, 95Y, 95M, 95V, 97A, 97W, 107M, 107K, 107H, 114Y, 114F, 114W, 114M, 133R, 133G, 133I, 167S, 167T, 167Y, 179C, 179M, 179T, 202P, 202W, 218T, 218H, 218V, 219A, 219N, 219R ; and at least one amino-acid modification on the alpha sub-unit chosen from the following list: 49H, 49W, 68G, 68T, 112V, 112P, 174K, 174L, 174C, 187A, 187G, 187V.

The numbers correspond to an amino-acid residue at position « XXX » (a number comprising 1, 2 or 3 digits) counted downstream from the N-terminal amino-acid residue in the amino-acid sequence of a β sub-unit or the alpha sub-unit.

Possible combinations are listed below. AA means amino-acid, and the common-used abbreviations for amino-acids are used such as A for Alanine. If a position is not mentioned the amino-acid is the amino-acid of the wild type of nitrile hydratase. For example, position β15 is not mentioned in the following combinations, it means that the amino-acid on position β15 is the amino-acid of the wild type amino-acid in position β15. The following combinations compri se the modification α174L but the description also comprises the same combinations in which the modification α174L is replaced by the modifications α174K or α174C.

### List of combinations

α174L, β17G, β46T, β48N, β57M, β95V, β114Y, β167S, β218H, β219A
α174L, β17G, β48W, β57M, β95V, β114Y, β167S, β218H, β219A
α174L, β17G, β48N, β57M, β95V, β114Y, β167S, β218H, β219A
α174L, β17G, β48N, β57M, β95V, β114F, β167S, β218H, β219A
α174L, β17G, β48N, β57M, β95V, β114Y, β167T, β218H, β219A
α174L, β17G, β48N, β57M, β95V, β114Y, β167S, β218H, β219A
α174L, β17G, β48N, β57M, β95V, β114Y, β167T, β218H, β219A
α174L, β57M, β95V, β114Y, β167S, β218H, β219A
α174L, β57M, β95V, β114F, β167S, β218H, β219A
α174L, β46T, β57M, β95V, β114Y, β167S, β218H, β219A
α174L, β46T, β48N, β57M, β95V, β114Y, β167S, β218H, β219A
α174L, β46T, β48N, β57M, β95V, β114Y, β167S, β218H, β219R
α174L, β46T, β48N, β57M, β95V, 0114W, 0167S, β218H, (3219R
α174L, β46T, β48N, β57M, β95V, β114Y, β167S, β218H, β219A
α174L, β17G, β46T, β48N, β57K, β95V, β114Y, β167S, β218H, β219A
α174L, β17G, β46T, β48N, β57K, β95V, β114Y, β167S, β218H, β219A
α174L, β17G, β46T, β48N, β57K, β95V, β114Y, β167S, β218H, β219R
α174L, β17G, β46T, β48N, β57K, β95V, β114Y, β167S, β218H, β219R
α174L, β17X, β48N, β57M, β95V, β114F, β167S, β218H, β219A
in the whole description, X is A or V,
α174L, β17X, β48N, β57M, β95V, β114F, β167S, β218H, β219A
α174L, β17X, β48N, β57M, β95V, β114F, β167S, β218H, β219R
α174L, β17X, β48N, β57M, β95V, β114F, β167S, β218H, β219R
α174L, β17X, β46T, β48N, β57M, β95V, β114F, β167S, β218H, β219R
α174L, β17X, β46T, β48N, β57M, β95V, β114F, β167S, β218H, β219A
α174L, β17X, β46T, β48N, β57M, β95V, β114F, β167S, β218H, β219A
α174L, β17X, β46T, β48N, β57K, β95Y, β114W, β167S, β218H, β219A
α174L, β17X, β46T, β48N, β57K, β95V, β114F, β167S, β218A, β219A
α174L, β17X, β46T, β48N, β57K, (395V, 0114F, 0167S, 0218Q, β219A
α174L, β17X, β46T, β48N, β57K, β95V, β114F, β167S, β218H, β219R
α174L, β17X, β46T, β48N, β57K, β95V, β107K, β114F, β167S, β218H, β219R
α174L, β17X, β48N, β57K, β95Y, β107K, β114W, β167S, β218H, β219A
α174L, β17X, β48N, β57M, β95V, β107K, β114W, β167S, β218H, β219A
α174L, β17X, β46T, β48N, β57M, β95V, β114W, β167S, β218H, β219A
α174L, β17X, β46T, β48N, β57K, β95V, β114W, β167S, β218H, β219A
α174L, β17X, β46T, β48N, β57M, β95V, β114W, β167S, β218H, β219R
α174L, β17X, β46T, β48N, β57K, β95V, β114W, β167R, β218H, β219R
α174L, β17X, β48N, β57K, β95Y, β114W, β167S, β218H, β219R
α174L, β17G, β46T, β48N, β57M, β95V, β107K, β114Y, β167S, β218H, β219A
α174L, β17G, β57M, β95V, β107L, β114Y, β167S, β218H, β219A
α174L, β17G, β48N, β57M, β95V, β107K, β114Y, β167S, β218H, β219A
α174L, β17G, β48N, β57M, β95V, β107K, β114F, β167S, β218H, β219A
α174L, β17G, β48N, β57M, β95V, β107K, β114Y, β167T, β218H, β219A
α174L, β17G, β48N, β57M, β95V, β107L, β114Y, β167S, β218H, β219A
α174L, β17G, β48N, β57M, β95V, β107L, β114Y, β167T, β218H, β219A
α174L, β57M, β95V, β107K, β114Y, β167S, β218H, β219A
α174L, β57M, β95V, β107K, β114F, β167S, β218H, β219A
α174L, β46T, β57M, β95V, β107K, β114Y, β167S, β218H, β219A
α174L, β46T, β48N, β57M, β95V, β107K, β114Y, β167S, β218H, β219A
α174L, β46T, β48N, β57M, β95V, β107K, β114Y, β167S, β218H, β219R
α174L, β46T, β48N, β57M, β95V, β107K, β114W, β167S, β218H, β219R
α174L, β46T, β48N, β57M, β95V, β107K, β114Y, β167S, β218H, β219A
α174L, β17G, β46T, β48N, β57K, β95V, β107K, β114Y, β167S, β218H, β219A
α174L, β17G, β46T, β48N, β57K, β95V, β107K, β114Y, β167S, β218H, β219A
α174L, β17G, β46T, β48N, β57K, β95V, β107K, β114Y, β167S, β218H, β219R
α174L, β17G, β46T, β48N, β57K, β95V, β107K, β114Y, β167S, β218H, β219R
α174L, β17X, β48N, β57M, β95V, β107K, β114F, β167S, β218H, β219A
α174L, β17X, β48N, β57M, β95V, β107K, β114F, β167S, β218H, β219A
α174L, β17X, β48N, β57M, β95V, β107K, β114F, β167S, β218H, β219R
α174L, β17X, β48N, β57M, β95V, β107K, β114F, β167S, β218H, β219R
α174L, β17X, β46T, β48N, β57M, β95V, β107K, β114F, β167S, β218H, β219R
α174L, β17X, β46T, β48N, β57M, β95V, β107K, β114F, β167S, β218H, β219A
α174L, β17X, β46T, β48N, β57M, β95V, β107H, β114F, β167S, β218H, β219A
α174L, β17X, β46T, β48N, β57K, β95Y, β107K, β114W, β167S, β218H, β219A
α174L, β17X, β46T, β48N, β57K, β95V, β107K, β114F, β167S, β218A, β219A
α174L, β17X, β46T, β48N, β57K, β95V, β107K, β114F, β167S, β218Q, β219A
α174L, β17X, β46T, β48N, β57K, β95V, β107K, β114F, β167S, β218H, β219R
α174L, β17X, β46T, β48N, β57K, β95V, β107K, β114F, β167S, β218H, β219R
α174L, β17X, β48N, β57K, β95Y, β107K, β114W, β167S, β218H, β219A
α174L, β17X, β48N, β57M, β95V, β107K, β114W, β167S, β218H, β219A
α174L, β17X, β46T, β48N, β57M, β95V, β107K, β114W, β167S, β218H, β219A
α174L, β17X, β46T, β48N, β57K, β95Y, β107K, β114W, β167S, β218H, β219A
α174L, β17X, β46T, β48N, β57M, β95V, β107K, β114W, β167S, β218H, β219R
α174L, β17X, β46T, β48N, β57K, β95Y, β107K, β114W, β167R, β218H, β219R
α174L, β17X, β48N, β57K, β95Y, β107K, β114W, β167S, β218H, β219R
α174L, β14S, β17G, β46T, β48N, β57M, β95V, β107K, β114Y, β167S, β218H, β219A
α174L, β14S, β17G, β57M, β95V, β107L, β114Y, β167S, β218H, β219A
α174L, β14S, β17G, β48N, β57M, β95V, β107K, β114Y, β167S, β218H, β219A
α174L, β14S, β17G, β48N, β57M, β95V, β107K, β114F, β167S, β218H, β219A
α174L, β14S, β17G, β48N, β57M, β95V, β107K, β114Y, β167T, β218H, β219A
α174L, β14S, β17G, β48N, β57M, β95V, β107L, β114Y, β167S, β218H, β219A
α174L, β14S, β17G, β48N, β57M, β95V, β107L, β114Y, β167T, β218H, β219A
α174L, β14S, β57M, β95V, β107K, β114Y, β167S, β218H, β219A
α174L, 014S, β57M, β95V, β107K, β114F, β167S, β218H, β219A
α174L, β14S, β46T, β57M, β95V, β107K, β114Y, β167S, β218H, β219A
α174L, β14S, β46T, β48N, β57M, β95V, β107K, β114Y, β167S, β218H, β219A
α174L, β14S, β46T, β48N, β57M, β95V, β107K, β114Y, β167S, β218H, β219R
α174L, β14S, β46T, β48N, β57M, β95V, β107K, β114W, β167S, β218H, β219R
α174L, β14S, β46T, β48N, β57M, β95V, β107K, β114Y, β167S, β218H, β219A
α174L, β14S, β17G, β46T, β48N, β57K, β95V, β107K, β114Y, β167S, β218H, β219A
α174L, β14S, β17G, β46T, β48N, β57K, β95V, β107K, β114Y, β167S, β218H, β219A
α174L, β14S, β17G, β46T, β48N, β57K, β95V, β107K, β114Y, β167S, β218H, β219R
α174L, β14S, β17G, β46T, β48N, β57K, β95V, β107K, β114Y, β167S, β218H, β219R
α174L, β14S, β17X, β48N, β57M, β95V, β107K, β114F, β167S, β218H, β219A
α174L, β14S, β17X, β48N, β57M, β95V, β107K, β114F, β167S, β218H, β219A
α174L, β14S, β17X, β48N, β57M, β95V, β107K, β114F, β167S, β218H, β219R
α174L, β14S, β17X, β48N, β57M, β95V, β107K, β114F, β167S, β218H, β219R
α174L, β14S, β17X, β46T, β48N, β57M, β95V, β107K, β114F, β167S, β218H, β219R
α174L, β14S, β17X, β46T, β48N, β57M, β95V, β107K, β114F, β167S, β218H, β219A
α174L, β14S, β17X, β46T, β48N, β57M, β95V, β107H, β114F, β167S, β218H, β219A
α174L, β14S, β17X, β46T, β48N, β57K, β95Y, β107K, β114W, β167S, β218H, β219A
α174L, β14S, β17X, β46T, β48N, β57K, β95V, β107K, β114F, β167S, β218A, β219A
α174L, β17X, β43Q, β46T, β48N, β57K, β95V, β107K, β114F, β167S, β218Q, β219A
α174L, β17X, β43Q, β46T, β48N, β57K, β95V, β107K, β114F, β167S, β218H, β219R
α174L, β17X, β43Q, β46T, β48N, β57K, β95V, β107K, β114F, β167S, β218H, β219R
α174L, β17X, β43Q, β48N, β57K, β95Y, β107K, β114W, β167S, β218H, β219A
α174L, β17X, β43Q, β48N, β57M, β95V, β107K, β114W, β167S, β218H, β219A
α174L, β17X, β43Q, β46T, β48N, β57M, β95V, β107K, β114W, β167S, β218H, β219A
α174L, β17X, β43Q, β46T, β48N, β57K, β95Y, β107K, β114W, β167S, β218H, β219A
α174L, β17X, β43Q, β46T, β48N, β57M, β95V, β107K, β114W, β167S, β218H, β219R
α174L, β17X, β43Q, β46T, β48N, β57K, β95Y, β107K, β114W, β167R, β218H, β219R
α174L, β17X, β43Q, β48N, β57K, β95Y, β107K, β114W, β167S, β218H, β219R
α174L, β14S, β17X, β46T, β48N, β57M, β95V, β107H, β114F, β167S, β218H, β219A
α174L, β14S, β17X, β46T, β48N, β57K, β95Y, β107K, β114W, β167S, β218H, β219A
α174L, β14S, β17X, β46T, β48N, β57K, β95V, β107K, β114F, β167S, β218A, β219A
α174L, β17X, β43Q, β46T, β48N, β57K, β77P, β95V, β107K, β114F, β167S, β218Q, β219A
α174L, β17X, β43Q, β46T, β48N, β57K, β77P, β95V, β107K, β114F, β167S, β218H, β219R
α174L, β17X, β43Q, β46T, β48N, β57K, β77P, β95V, β107K, β114F, β167S, β218H, β219R
α174L, β17X, β43Q, β48N, β57K, β77P, β95Y, β107K, β114W, β167S, β218H, β219A
α174L, β17X, β43Q, β48N, β57M, β77P, β95Y, β107K, β114W, β167S, β218H, β219A
α174L, β17X, β43Q, β46T, β48N, β57M, β77P, β95Y, β107K, β114W, β167S, β218H, β219A
α174L, β14S, β46T, β48N, β57M, β97A, β107K, β114W, β1675, β218H, β219R
α174L, β14S, β46T, β48N, β57M, β97A, β107K, β114Y, β167S, β218H, β219A
α174L, β14S, β17G, β46T, β48N, β57K, β97A, β107K, β114Y, β167S, β218H, β219A
α174L, β14S, β46T, β48N, β57K, β97A, β107K, β114Y, β167S, β218H, β219A
α174L, β14S, β17G, β46T, β48N, β57K, β97A, β107K, β114Y, β167S, β218H, β219R
α174L, β14S, β17G, β46T, β48N, β57K, β97A, β107K, β114Y, β167S, β218H, β219R
α174L, β14S, β17X, β48N, β57M, β97A, β107K, β114F, β167S, β218H, β219A
α174L, β14S, β17X, β48N, β57M, β97A, β107K, β114F, β167S, β218H, β219A
α174L, β14S, β17X, β48N, β57M, β97A, β107K, β114F, β167S, β218H, β219R
α49H, β17G, β48N, β57M, β95V, β114Y, β167T, β218H, β219A
α49H, β17G, β48N, β57M, β95V, β114Y, β167S, β218H, β219A
α49H, β17G, β48N, β57M, β95V, β114Y, β167T, β218H, β219A
α49H, β57M, β95V, β114Y, β167S, β218H, β219A
α49H, β57M, β95V, β114P, β1678, β218H, β219A
α49H, β46T, β57M, β95V, β114Y, β1678, β218H, β219A
α49H, β46T, β48N, β57M, β95Y, β114Y, β1678, β218H, β219A
α49H, β46T, β48N, β57M, β95Y, β114Y, β1678, β218H, β219R
α49H, β46T, β48N, β57M, β95V, β114W, β167S, β218H, β219R
α49H, β17X, β46T, β48N, β57M, β95Y, β114F, β1678, β218H, β219R
α49H, β17X, β46T, β48N, β57M, β95Y, β114F, β1678, β218H, β219A
α68T, β17X, β46T, β48N, β57M, β95Y, β114F, β1678, β218H, β219A
α68T, β17X, β46T, β48N, β57K, β95V, β114W, β167S, β218H, β219A
α68T, β17X, β46T, β48N, β57K, β95V, β114F, β1678, β218A, β219A
α68T, β17X, β46T, β48N, β57K, β95V, β114F, β1678, β218Q, β219A
α68T, β17X, β46T, β48N, β57K, β95V, β114F, β1678, β218H, β219R
α68T, β17X, β46T, β48N, β57K, β95V, β107K, β114F, β167S, β218H, β219R
α68T, β46T, β48N, β57M, β95V, β107K, β114W, β167S, β218H, β219R
α68T, β46T, β48N, β57M, β95V, β107K, β114Y, β1678, β218H, β219A
α68G, β17G, β46T, β48N, β57K, β95V, β107K, β114Y, β1678, β218H, β219A
α68G, β17G, β46T, β48N, β57K, β95V, β107K, β114Y, β1678, β218H, β219A
α68G, β17G, β46T, β48N, β57K, β95V, β107K, β114Y, β1678, β218H, β219R
α68G, β17G, β46T, β48N, β57K, β95V, β107K, β114Y, β1678, β218H, β219R
α68G, β17X, β48N, β57M, β95V, β107K, β114F, β1678, β218H, β219A
α68G, β17X, β46T, β48N, β57M, β95V, β107K, β114F, β167S, β218H, β219R
α112V, β17X, β46T, β48N, β57M, β95V, β107K, β114F, β1678, β218H, β219A
α112V, β17X, β46T, β48N, β57M, β95Y, β107H, β114F, β1678, β218H, β219A
α112V, β17X, β46T, β48N, β57K, β95Y, β107K, β114W, β1678, β218H, β219A
α112V, β17X, β46T, β48N, β57K, β95V, β107K, β114F, β1678, β218A, β219A
α112V, β17X, β46T, β48N, β57K, β95V, β107K, β114F, β1678, β218Q, β219A
α112V, β17X, β46T, β48N, β57K, β95V, β107K, β114F, β167S, β218H, β219R
α187G, β17X, β46T, β48N, β57K, β95V, β107K, β114F, β167S, β218H, β219R
α187G, β17X, β48N, β57K, β95V, β107K, β114W, β1678, β218H, β219A
α187G, β17X, β48N, β57M, β95V, β107K, β114W, β1678, β218H, β219A
α187G, β14S, β46T, β48N, β57M, β95V, β107K, β114W, β167S, β218H, β219R
α187G, β14S, β46T, β48N, β57M, β95V, β107K, β114Y, β1678, β218H, β219A
α187V, β14S, β17G, β46T, β48N, β57K, β95V, β107K, β114Y, β1678, β218H, β219A
α187V, β14S, β17G, β46T, β48N, β57K, β95V, β107K, β114Y, β1678, β218H, β219A
α187V, β14S, β17X, β46T, β48N, β57K, β95V, β107K, β114F, β167S, β218A, β219A
α187V, β17X, β43Q, β46T, β48N, β57K, β95V, β107K, β114F, β167S, β218Q, β219A
α187V, β17X, β43Q, β46T, β48N, β57K, β95V, β107K, β114F, β167S, β218H, β219R
α187V, β17X, β43Q, β46T, β48N, β57K, β95V, β107K, β114F, β167S, β218H, β219R
α187V, β17X, β43Q, β48N, β57K, β95V, β107K, β114W, β167S, β218H, β219A
α187V, β17X, β43Q, β48N, β57M, β95Y, β107K, β114W, β167S, β218H, β219A
α187V, β17X, β43Q, β46T, β48N, β57M, β95Y, β107K, β114W, β167S, β218H, β219A
α187V, β17X, β43Q, β46T, β48N, β57K, β95V, β107K, β114W, β167S, β218H, β219A

In one embodiment, the amount of the biocatalyst is 0.0001 kg dry cells/m³ to 2 kg dry cells/m³ of reaction mixture.

In some embodiments, the biocatalyst comprises 0.0001 to 0.2 kg dry cells/m³ of the reaction mixture and the biocatalyst is a microbe selected from the group consisting of *Rhodococcus, Aspergillus, Acidovorax, Agrobacterium, Bacillus, Bradyrhizobium, Burkholderia, Escherichia, Geobacillus, Klebsiella, Mesorhizobium, Moraxella, Pantoea, Pseudomonas, Rhizobium, Rhodopseudomonas, Serratia, Amycolatopsis, Arthrobacter, Brevibacterium, Corynebacterium, Microbacterium, Micrococcus, Nocardia, Pseudonocardia, Trichoderma, Myrothecium, Aureobasidium, Candida, Cryptococcus, Debaryomyces, Geotrichum, Hanseniaspora, Kluyveromyces, Pichia, Rhodotorula, Comomonas,* and *Pyrococcus,* more preferably, the biocatalyst is selected from the group consisting of *Rhodococcus, Pseudomonas, Escherichia* and *Geobacillus* or a combination of at least two of any of the foregoing.

During the process, more of the biocatalyst may be added, for example, if acrylonitrile starts to accumulate in the reactor. The biocatalyst may be added, for example, as a homogeneous slurry in water. Biocatalyst can be added at any point of the process.

### 4. Reaction Progression

The reaction is conducted at atmospheric pressure, more specifically at 1 bar absolute.

The slurry may be produced by any known method in the art, such as mixing water and the biocatalyst in a receptacle or in the reactor. More specifically the slurry is homogeneous. Strongly agglomerated slurry is less active than homogenous slurry. The biocatalyst is more active in homogenous slurry.

A reaction of acrylonitrile to acrylamide in aqueous solution in the presence of biocatalyst having NHase activity begins once acrylonitrile is fed into a reactor comprising said slurry. Thus, the feeding of acrylonitrile into a reactor comprising said slurry provides a reaction mixture comprising water, acrylamide, acrylonitrile, and biocatalyst.

The acrylamide production can be done in a discontinuous or a continuous process. The reactor combination can be from 1 to 50 reactors, more specifically from 1 to 20, more specifically 1-10 with a distribution in series and/or partially in parallel, more specifically in series. The reactor can be a combination of stirred tank reactor, unstirred reactor tank, plug-flow reactor. The reactor size can be from 0.0001 m³ to 100 m³; 0.0001 m³ to 50 m³ and more specifically from 5 to 40m³. In the case of a continuous process, the reactants addition rates are continuously monitored in the first reactor or a combination in the different reactors. The global flowrate is adjusted to reach a residence time between 0.1 - 20h, 0.5-15h, 1-10h, specifically 3-8h. The reactants are listed as the acrylonitrile, the biocatalyst, the water, any organic or mineral salt solution, any other additives (for example MEHQ acting as a polymerization inhibitor). The last reactors are generally used for maturation only without additional reactant addition. In the case of a discontinuous process the acrylonitrile additions are done subsequently using different flowrate from low to high rate, a reverse or a combination. In each reactor the pH is regulated at a defined value between 6 to 9, each reactor can be monitored at different value using an acid or base addition, the acid and base can be organic or mineral. The temperature is regulated in each reactor using an internal or external cooling/heating system. At process end the biocatalyst (free cell or with a support) can be separated from the aqueous acrylamide solution using well known separative techniques: filtration, centrifugation.

An aqueous solution of acrylamide in high concentration (e.g. at least 20 wt%, or at least 30 wt% or at least 40 wt%, or at least 45 wt%, or at least 50 wt% or more) can be produced with controlled acrylonitrile feed and process temperature profiles. Cooling of the reactor is typically needed to keep the reaction mixture at a desired reaction temperature. The temperature and the acrylonitrile feed rate are each relatively high at the beginning of the reaction to achieve a fast reaction rate and short synthesis time. The acrylonitrile feed rate is relatively low during the last hours to avoid acrylonitrile accumulation in the reactor.

The feeding of acrylonitrile may be continued throughout the process, more specifically continued throughout the process until the maturation (aging) phase. Feed rate of the acrylonitrile may vary during the process. The feeding of acrylonitrile may be continuous or intermittent. The feed rate of acrylonitrile depends on the reaction rate of the acrylonitrile to acrylamide and the rate of biocatalyst deactivation. In one embodiment, feeding of the acrylonitrile is continued throughout the process until the maturation phase.

In one embodiment the acrylonitrile feed rate is adjusted during the process to avoid acrylonitrile accumulation into the reaction mixture. The acrylonitrile is fed during the process with such a rate at which the acrylonitrile converts to acrylamide. More specifically the acrylonitrile amount in the reaction mixture is maintained as less than 5 wt%, or less than 2.5 wt%, more specifically less than 1 wt%, even more specifically less than 0.5 wt% relative to the total amount of reaction mixture.

In some embodiments, the acrylonitrile feed rate is adjusted during the process, thereby controlling acrylonitrile accumulation in the reactor headspace; and 38 % to 48 % of the total amount of acrylonitrile fed to the reactor is fed during the time period spanning 0 min to 60 min inclusive from the beginning of feeding acrylonitrile into the reactor.

In another embodiment of the process, 30 % to 60 % of total amount of acrylonitrile fed to the reactor is fed during 0 min to 100 min from the beginning of the process; 10 % to 40 % of total amount of acrylonitrile is fed between 100 min to 220 min of the process; 5% to 20 % of total amount of acrylonitrile is fed between 220 min to 360 min of the process. To reach balance of 100% of fed acrylonitrile, the rest acrylonitrile is fed during the process prior to the maturation phase.

During the maturation phase, substantially no acrylonitrile, is fed to the reactor. During maturation, acrylonitrile monomers still present in the reaction mixture react to acrylamide. The reaction mixture is maturated until desired features are reached.

It is known that the biocatalyst starts to deactivate in around 25 wt% to 38 wt% acrylamide solution. See, for example, WO2019/097123. Biocatalyst deactivation caused by acrylamide accumulation is strongly dependent on temperature, and cooling of the reaction mixture notably reduces biocatalyst deactivation. As such, the temperature of the reaction mixture is monitored. The monitoring temperature and measuring may be performed with any suitable means and methods in the art.

Initially, the temperature of the reaction mixture is maintained at 15 to 35 °C. In one embodiment the temperature is maintained at 19 to 30 °C, more specifically at 20 to 28°C and even more specifically at 22 to 26 °C. In one embodiment, the temperature is maintained in the desired range by measuring the temperature of the reaction mixture and either cooling the mixture or heating the mixture so that the temperature stays in the desired range. The cooling and/or heating of the reaction mixture may be conducted with known methods in the art.

In some embodiments, the process comprises further cooling of the reaction mixture when the acrylamide concentration reaches at least 10 wt%, more specifically 10 wt% to 38 wt%.

In one embodiment the cooling of said reaction mixture is started when the acrylamide concentration reaches 28 wt% to 30 wt%. The cooling of the reaction mixture can be performed by any suitable method and means known in the art, such as by cooling the reactor. When the cooling of the reaction mixture is started, the temperature of the reaction mixture may be the same, higher, or lower than the temperature of the reaction mixture in the beginning of the process.

In some embodiments, cooling of the reaction mixture is continued so that when the acrylamide concentration reaches 35 wt% to 55 wt%, the temperature of the reaction mixture is within a range of 10 °C to 30 °C, or 15 °C to 27 °C. In other words, the time period of the cooling of the reaction mixture to the temperature of 10 °C to 30°C, or 15 °C to 27 °C, is the time period when the acrylamide concentration of at least 27 wt% (more specifically 27 wt% to 38 wt%) increases to acrylamide concentration 35 wt% to 55 wt% (more specifically 40 wt% to 50 wt%).

In one embodiment, the cooling of the reaction mixture is continued so that so that when the acrylamide concentration reaches 35 wt% to 55 wt%, the temperature is within a range of 10 °C to 30 °C, more specifically 18 °C to 27 °C, and even more specifically, the temperature is 22-26 °C. In one embodiment the cooling is started, for example, after the reaction mixture has been maintained at 15 °C to 25 °C.

In one embodiment, the reaction mixture is maturated at a temperature within a range of 15 °C to 25 °C, or 18 °C to 27 °C when the acrylamide concentration reaches 35 wt% to 55 wt%.

During the maturation phase, substantially no acrylonitrile, and more specifically no acrylonitrile, is fed to the reactor. During maturation phase, unreacted acrylonitrile in the reactor reacts to acrylamide. Maturation begins after the reaction mixture has been cooled and the temperature of the reaction mixture is within the range of 15 °C to 25°C, or 15 °C to 27 °C, and/or after the feeding of acrylonitrile into the reactor has ended. More specifically the maturation is continued until final concentration of the acrylonitrile in reactor headspace is at most 10000 ppmv, at most 2500 ppmv, at most 1000 ppmv, at most 250 ppmv, or at most 100 ppmv.

In one embodiment of the process, the temperature of the reaction mixture is maintained at 15 °C to 25 °C for 30 min to 90 min, preferably for 45 min to 60 min and the cooling of the reaction mixture to the temperature of 10 °C to 30 °C, or 15 °C to 27 °C is performed during a period of time of 45 min to 120 min, preferably for 60 min to 120 min.

As the temperature is kept low at end of the process, less acrylic acid is formed in the process. The activation energy of a reaction forming acrylic acid is higher than the activation energy of the main reaction (formation of acrylamide). The amount of acrylic acid in the aqueous acrylamide solution is at most 300 ppm, more specifically at most 200 ppm, even more specifically at most 100 ppm. Low amount of acrylic acid in the aqueous acrylamide solution is advantageous when cationic or non-ionic polymers are prepared from the acrylamide solution.

The produced aqueous acrylamide solution may be filtered to separate acrylamide from biocatalyst. The filtration can be done using various filtration process as centrifuge filtration, vacuum or pressure filtration. The filtration process can be continuous or discontinuous. A variety of filtration medium is possible as diatomaceous, perlite, cellulose or any micrometric filter cloth.

### 5. Aqueous Acrylamide Solution

In a second aspect of the present disclosure there is provided an aqueous acrylamide solution obtained or obtainable by the process disclosed herein. More particularly there is provided an aqueous acrylamide solution obtained by the process disclosed herein and characterized in that a concentration of total residual acrylonitrile in the aqueous acrylamide is obtained by analysis of acrylonitrile in reactor headspace and is equal to or less than 300 ppmv, as measured by online-GC chromatography.

In some embodiments, the turbidity of the aqueous acrylamide solution may be equal to or less than 20 NTU as measured by absorbance at 450 nm of a mixture comprising 0.7 ml HCl (0.1 N), 7 ml acetone, and 2.3 ml filtered (0.45 µm) aqueous acrylamide sample. In one embodiment the turbidity of the solution is equal to or less than 15.

In some embodiments, the produced aqueous acrylamide solution may be substantially free of biocatalyst.

### 6. Use of Aqueous Acrylamide Solution

In a third aspect of the present disclosure, there is provided use of the aqueous acrylamide solution produced by the disclosed process in manufacturing of polyacrylamides. Polyacrylamides are water-soluble polymers or water swellable polymers containing at least acrylamide monomer.

Water-soluble polymer is meant a polymer which gives an aqueous solution without insoluble particles when dissolved with stirring at 25°C and with a concentration of 10 g.L⁻¹ in deionized water.

Water swellable polymers are cross-linked polymers which form three-dimensional networks. These water-swellable polymers are also known as super-absorbent polymers. Generally, they have a water absorption capacity of more than 10 times their volume.

Polyacrylamides are synthetic polymers and more preferably they contain at least one non-ionic different form acrylamide and/or anionic and/or cationic hydrophilic monomers chosen in the following list:
- nonionic monomers: acrylonitrile, methacrylamide, N-vinylformamide (NVF), N-vinylacetamide, N-vinylpyrrolidone (NVP), N-vinylimidazole, N-vinyl succinimide, acryloyl morpholine (ACMO), glycidyl methacrylate, glyceryl methacrylate, diacetone acrylamide, N-methylolacrylamide (NMA), hydroxyalkyl(C₁-C₃)-(meth)acrylate, thioalkyl (C₁-C₃)-(meth)acrylate and mixtures thereof,
- anionic monomers: acrylic acid, methacrylic acid, itaconic acid, crotonic acid, maleic acid, fumaric acid, acrylamido undecanoic acid, 3-acrylamido 3-methylbutanoic acid, maleic anhydride, 2-acrylamido-2-methylpropane sulfonic acid (ATBS), vinylsulfonic acid, vinylphosphonic acid, methallylphophonic acid, 2-sulfoethylmethacrylate, sulfopropylmethacrylate, sulfopropylacrylate, allylphosphonic acid, styrene sulfonic acid, 2-acrylamido-2-methylpropane disulfonic acid, their salts and mixtures thereof,
- cationic monomers: diallyldialkyl ammonium salts, such as diallyl dimethyl ammonium chloride (DADMAC); acidified or quaternized salts of dialkylaminoalkylacrylamides; acidified or quaternized salts of dialkyl-aminoalkylmethacrylamides, e.g. methacrylamidopropyl trimethyl ammonium chloride (MAPTAC), acrylamido-propyl trimethyl ammonium chloride (APTAC), acidified or quaternized salts of dialkylaminoalkyl acrylate, such as quaternized or salified dimethylaminoethyl acrylate (ADAME), acidified or quaternized salts of dialkylaminoalkyl methacrylate, such as quaternized or salified dimethylaminoethyl methacrylate (MADAME), and mixtures thereof. Alkyl groups are C₁-C₃.

Optionally polyacrylamides contain zwitterionic hydrophilic monomer chosen in the list: dimethylaminoethyl acrylate derivatives, such as 2-((2-(acryloyloxy)ethyl) dimethylammonio) ethane-1-sulfonate, 3-((2-(acryloyloxy)ethyl) dimethylammonio) propane-1-sulfonate, 4-((2-(acryloyloxy)ethyl) dimethylammonio) butane-1-sulfonate, [2-(acryloyloxy)ethyl] (dimethylammonio) acetate, dimethylaminoethyl methacrylate derivatives such as 2-((2-(methacryloyloxy)ethyl)dimethylammonio)ethane-1-sulfonate, 3-((2-(methacryloyloxy) ethyl) dimethylammonio) propane-1-sulfonate, 4-((2-(methacryloyloxy) ethyl) dimethylammonio) butane-1-sulfonate, [2-(methacryloyloxy)ethyl] (dimethylammonio) acetate, dimethylamino propylacrylamide derivatives such as 2-((3-acrylamidopropyl) dimethylammonio) ethane-1-sulfonate, 3-((3-acrylamidopropyl) dimethylammonio) propane-1-sulfonate, 4-((3-acrylamidopropyl) dimethylammonio) butane-1-sulfonate, [3-(acryloyloxy) propyl] (dimethylammonio) acetate, dimethylamino propyl methylacrylamide derivatives such as 2-((3-methacrylamidopropyl) dimethylammonio) ethane-1-sulfonate, 3-((3-methacrylamidopropyl) dimethylammonio) propane-1-sulfonate, 4-((3-methacrylamidopropyl) dimethylammonio) butane-1-sulfonate and [3-(methacryloyloxy)propyl] (dimethylammonio) acetate and mixtures thereof.

Optionally polyacrylamides contain hydrophobic monomers chosen in the list: groups consisting of (meth)acrylic acid esters with a C₄-C₃₀ alkyl, arylalkyl (C₄-C₃₀ alkyl, C₄-C₃₀ aryl), propoxylated, ethoxylated or ethoxylated and propoxylated chain; (meth)acrylamide derivatives with a propoxylated, ethoxylated, ethoxylated and propoxylated C₁-C₃ alkyl, arylalkyl (C₄-C₃₀ alkyl, C₄-C₃₀ aryl) or dialkyl (C₄-C₃₀ alkyl) chain; alkyl aryl sulfonates (C₄-C₃₀ alkyl, C₄-C₃₀ aryl), or by mono- or di-substituted (meth)acrylamide amides having a C₄-C₃₀ alkyl, arylalkyl (C₄-C₃₀ alkyl, C₄-C₃₀ aryl), propoxylated, ethoxylated, or ethoxylated and propoxylated chain; (meth)acrylamide derivatives with a C₄-C₃₀ alkyl, propoxylated arylalkyl (C₄-C₃₀ alkyl, C₄-C₃₀ aryl), ethoxylated, ethoxylated and propoxylated, or C₄-C₃₀ dialkyl chain; alkyl aryl sulfonates (C₄-C₃₀ alkyl, C₄-C₃₀ aryl) and mixtures thereof.

Water-soluble polyacrylamides advantageously comprise less than 1 mol% hydrophobic monomers. It may be devoid of hydrophobic monomers.

Water soluble polyacrylamides are advantageously linear or structured. Structured polymer refers to a non-linear polymer which has side chains to obtain, when this polymer is dissolved in water, a strong state of entanglement leading to very high low-gradient viscosities.

The water-soluble polymer according to the invention can further be structured:
- by at least one structuring agent, which may be selected from the group comprising polyethylenically unsaturated monomers (having at least two unsaturated functions), such as vinyl functions, in particular allyl functions,
   acrylic and epoxy functions, such as methylene bis acrylamide (MBA), triallylamine, or tetraallylammonium chloride or 1,2 dihydroxyethylene bis-(N-acrylamide), and/or
- macroinitiators such as polyperoxides, polyazoids and polyagents such as transfer agents such as polymer-capturing (co)polymers and polyols, and/or
- functionalized polysaccharides.

According to the invention, the water-soluble polyacrylamide can have a linear, branched, star, comb, dendritic or block structure. These structures can be obtained by selecting the initiator, transfer agent, polymerization technique, such as controlled radical polymerization known as RAFT (reversible addition fragmentation chain transfer), NMP (Nitroxide Mediated Polymerization") or the incorporation of structural monomers, the concentration...

Water swellable polyacrylamides are crosslinked with crosslinking agent which can be selected from polyethylenically unsaturated monomers (having at least two unsaturated functions) such as, for example, vinylic functions, notably allylic, acrylic functions, or from the monomers having at least two epoxy functions. One can mention, for example, methylenebisacrylamide (MBA), triallylamine, tetraallylammonium chloride, 1,2-dihydroxyethylenebis-(N-acrylamide) and the mixtures thereof. Preferably crosslinking agent is methylenebisacrylamide (MBA).

The quantity of crosslinking agent in water swellable polymers is advantageously between 5 and 5000 ppm with respect to the total weight of the monomers, more preferably between 100 and 1000 ppm.

Polyacrylamides do not require the development of any polymerization process. In fact, it can be obtained using any polymerization technique well known to those skilled in the art. It can be obtained by solution polymerization; gel polymerization; precipitation polymerization; emulsion polymerization (aqueous or inverse); suspension polymerization; polymer-reactive extrusion polymerization; water-in-water polymerization; or micellar polymerization.

Polymerization is generally free-radical polymerization. By free-radical polymerization, we include free-radical polymerization using UV, azo, redox, or thermal initiators, as well as controlled radical polymerization (CRP) techniques or matrix polymerization techniques.

Polyacrylamides to the invention can be modified after it has been obtained by polymerization. This is referred to as post-modification of the polymer. All known post-modifications can be applied to the polymer according to the invention. Preferred modification is post-hydrolysis.

Post-hydrolysis consists of the reaction of hydrolysable functional groups of monomer units, advantageously non-ionic, more preferably amide or ester functions, with a hydrolysis agent. This hydrolysis agent may be an enzyme, an ion exchange resin, an alkali metal or a suitable acidic compound. Preferably, the hydrolysis agent is a Bronsted base. When the polymer comprises monomeric amide and/or ester units ester monomer units, then the post-hydrolysis reaction produces carboxylate groups.

According to the invention, polyacrylamide may be in liquid, gel or solid form when its preparation includes a drying step such as spray drying, drum drying, radiation drying such as microwave drying, or fluidized bed drying.

The aqueous acrylamide solution obtained by the process of the invention can be used for the manufacture of polyacrylamides, or N-methylenebisacrylamide or N-methylolacrylamide or N,N'-methyleneacrylamido-2-methylacrylamide.

Polyacrylamides can be used in a field selected from hydrocarbon recovery; in well drilling and cementing; hydrocarbon well stimulation hydrocarbon wells; in water treatment; in the treatment of fermentation treatment; sludge treatment; fabrication; in construction; in wood treatment.
in hydraulic composition treatment; in the mining industry; in cosmetics formulation; in detergents formulation; in textile manufacturing; in the manufacture of battery components; in geothermal energy; in the manufacture of hygienic or in agriculture.

Polyacrylamides can be used as a flocculant, coagulant, binding agent, fixing agent, viscosity reducer, thickening agent, absorbent agent, friction reducer agent, dewatering agent, draining agent, charge retention agent, dehydrating agent, conditioning agent, stabilizing agent, fixing agent, film-forming agent, sizing agent, super plasticizing agent, clay inhibitor or dispersant.

### 7. Examples

The following examples are provided for illustrative purposes only and are non-limiting.

### Materials and Methods for Monitoring Acrylamide Synthesis Reaction Used in Examples

Simulation of acrylamide reaction has been performed in reactors to build mathematical model without having the need to use additional analytical reference technique, compared to NIR with either HPLC or GC.

Reactions were performed as described in Examples 1-4. Concentrations of acrylonitrile (AN) were determined by online-GC in reactor's headspace. The concentration of the acrylamide (AMD) solution is assessed through refractive index (RI) measurement. The weight percentage concentration is then determined by its correlation with the refractive index using a calibration curve. A few drops of the sample solution are placed on the refractometer prism, and the corresponding value is read. For instance, the Mettler Toledo RM40 refractometer can be utilized in this process. Additional GC offline analysis has been made to fully certify that the online instrument is giving the good concentrations.

Online-GC was performed using online instrument from Chromatotec.

**Table 1: Online GC parameters**

| Instrument | ChromaFID Online-GC | Perkin Elmer offline-GC |
|---|---|---|
| Chromatographic precolumn & column | MXT 30CE | PORAPAK PS 1m, 1/8" |
| | 1m*0.28mm*1µm | |
| | MXT 30CE 29m*0.28mm*1µm | |
| Detector | FID, 170°C | FID, 250°C |
| Sample loop | 40 - 125 µL deactivated MXT | 0,5 µL |
| Carrier gas | Hydrogen | Nitrogen |

For offline-GC measurements, reaction mixture samples (1.5 mL) were removed periodically from the reactor, filtered through a 0.2 µm RC syringe filter and quenched by addition of sulfuric acid.

Concentrations of AN were determined by offline-GC using an Clarus 590 (Perkin Elmer), equipped with aPORAPAK PS packed column, 1m, 1/8". Chemical was detected by FID. The flow rate was 25 mL/min with nitrogen as carrier gas. 0.5 µL of sample is injected at 200°C with oven at 170°C in isotherm. The samples for GC were prepared by accurately measuring about 0.5 g of quenched reaction mixture and diluting it into 10 ml of Type 1 Milli-Q water.

The online-GC values determined were correlated to offline GC to generate representative data between acrylonitrile in liquid acrylamide and acrylonitrile in reactor's headspace. Laboratory experiment has been conducted for the establishment of a correlation between acrylonitrile concentration in liquid (offline GC) versus acrylonitrile in reactor's headspace (online GC). Refractive index allowed to determine acrylamide concentration and select the proper most accurate mathematical model for online GC.

### Example 1: Acrylamide synthesis at lab scale

In 1 L glass reactor equipped with a double-jacket thermal regulated with an external circulator, a mechanical stirrer, 627 g deionized water is loaded. The stirring is started and set at 180 RPM. The pH of the solution is adjusted at a value of 8 by adding drops of a 10% sodium hydroxide solution. The medium temperature is adjusted to 18°C. 500 µL of a biocatalyst NHase 9%wt solution prepared from Rhodococcus rhodochrous J-1 strain, with an activity of 880 U, is added (the unit U means to produce 1 micromolar amide compound from the nitrile compound in 1 minute). The temperature regulation is set to 20°C during the acrylonitrile additions and ageing steps. Successive acrylonitrile additions are done at a flowrate of 1.77 g/min for 105 min, then 1.03 g/min for 116 min and 0.54 g/min for 126 min. The reaction medium is let for 3h under continuous stirring. The reactor gas headspace is analyzed using the online method previously described. At the end of each acrylonitrile addition and every 1 hour until the final ageing, liquid samples are collected, prepared as described previously for refractive index measurement. The results are reported in Table 2. Off-line GC is used at process end to measure the residual acrylonitrile in the liquid phase, a value of 15 ppm is measured.

**Table 2: Concentrations measured during acrylamide reaction.**

| Sampling time (min) | RI measurement | AMD (wt%) | AN (ppmv) in reactor gas holdup |
|---|---|---|---|
| 105 | 1.3766 | 28.32 | 28166 |
| 221 | 1.3995 | 42.61 | 24263 |
| 348 | 1.4088 | 48.51 | 25842 |
| 408 | 1.4130 | 49.95 | 187 |
| 468 | 1.4135 | 50.8 | 115 |
| 527 | 1.4135 | 50.8 | 35 |

### Example 2: Acrylamide Experiment in industrial process

10 continuous reactors stirred are combined in series, 10 m3 each. The five first reactor are continuously fed with acrylonitrile, the reactor 1 is fed with the Nitrile hydratase catalyst as defined in the example 1 and water (table 3). The pH is continuously adjusted at a pH of 7.5 using a caustic solution à 10%wt in the reactor 1. The flowrate of the catalyst is continuously adjusted the flowrate are reported in table 4. The temperature is set at 25°C in each reactor.

**Table 3: Global process condition**

| Reactor number | R1 | R2 | R3 | R4 | R5 |
|---|---|---|---|---|---|
| AN flowrate (L/h) | 1390 | 745 | 391 | 404 | 173 |
| Catalyst (kg/h) | 2 to 4.0 | | | | |
| Water(kg/h) | 4100 | | | | |

The reactor headspace in reactor 8 is continuously measured using the online GC. The value is used to continuously adjust the catalyst flowrate. The catalyst adjustment is needed because of fluctuation on the acrylonitrile quality due to impurities content. The values are reported in the table 4.

**Table 4: acrylonitrile concentrations and catalyst flowrates**

| Sampling Date | Daily average Acrylonitrile determination (1 analysis every 15 minutes) in reactor 8 headspace, ppmv | Daily average catalyst flowrate (kg/h) |
|---|---|---|
| Day 1 | 2508 | 2,5 |
| Day 2 | 2043 | 2.8 |
| Day 3 | 2043 | 2.8 |
| Day 4 | 3465 | 2.0 |
| Day 5 | 1380 | 3.5 |
| Day 6 | 2079 | 2.8 |
| Day 7 | 1840 | 3.0 |
| Day 8 | 2270 | 2.9 |
| Day 9 | 1362 | 3.5 |
| Day 10 | 3405 | 2.0 |
| Day 11 | 1840 | 3.0 |
| Day 12 | 1392 | 3.5 |
| Day 13 | 696 | 3.5 |
| Day 14 | 1362 | 3.5 |
| Day 15 | 2519 | 2.7 |
| Day 16 | 2519 | 2.7 |
| Day 17 | 1872 | 3.0 |
| Day 18 | 1150 | 3.7 |
| Day 19 | 1170 | 3.7 |
| Day 20 | 1155 | 3.7 |
| Day 21 | 936 | 3.8 |
| Day 22 | 1589 | 3.2 |
| Day 23 | 1380 | 3.5 |
| Day 24 | 2736 | 2.7 |
| Day 25 | 3248 | 2.5 |

The acrylamide solution is centrifuged after the reactor 10 to remove the catalyst bacteria cells. The acrylonitrile residual is measured with the offline GC method. A value less than 100 ppm is always measured each day. The average catalyst consumption during the production campaign was 3.06 kg/h.

### Example 3: Acrylamide Experiment in industrial process, off-line analysis only

With the same industrial running condition as described in example 2 the acrylamide was synthetized except there is no online GC analysis in the reactor headspace but only an off-line GC analysis once a day in each reactor. The production day differed from example 2 and can lead to acrylonitrile quality variation.

The acrylonitrile value is used to adjust the catalyst flowrate every day. The values measured are reported in the table 5. The acrylamide solution is centrifuged after the reactor 10 to remove the catalyst bacteria cells to be used for polymerization.

In comparison to the example 2 the global catalyst consumption was larger; the average catalyst consumption during the production campaign was 3.93 kg/h, 28% higher than in Example 2. Furthermore, the acrylonitrile residual has exceeded occasionally 100 ppm in R10.

**Table 5:**

| Sampling Date | Acrylonitrile measurements (ppm) at 8 a.m | | | | | | | | | | Catalyst flowrate (kg/h) set at 8 a.m |
|---|---|---|---|---|---|---|---|---|---|---|---|
| | R1 | R2 | R3 | R4 | R5 | R6 | R7 | R8 | R9 | R10 | |
| Day 1 | 21175 | 18034 | 13616 | 10504 | 11605 | 8995 | 5997 | 2998 | 600 | 108 | 3.8 |
| Day2 | 24351 | 20739 | 15658 | 12080 | 13346 | 10344 | 6896 | 3448 | 690 | 124 | 4.1 |
| Day3 | 30439 | 25924 | 19573 | 15099 | 16682 | 12930 | 8620 | 4310 | 862 | 155 | 4.3 |
| Day4 | 23134 | 19702 | 14875 | 11476 | 12679 | 9827 | 6551 | 3276 | 655 | 118 | 4.1 |
| Day5 | 17119 | 14580 | 11008 | 8492 | 9382 | 7272 | 4848 | 2424 | 485 | 87 | 3.8 |
| Day6 | 18660 | 15892 | 11998 | 9256 | 10227 | 7927 | 5284 | 2642 | 528 | 125 | 4.1 |
| Day7 | 13062 | 11124 | 8399 | 6479 | 7159 | 5549 | 3699 | 1850 | 370 | 67 | 3.6 |
| Day8 | 16850 | 14350 | 10834 | 8358 | 9235 | 7158 | 4772 | 2386 | 477 | 86 | 3.9 |
| Day9 | 16007 | 13633 | 10293 | 7940 | 8773 | 6800 | 4533 | 2267 | 453 | 82 | 3.9 |
| Day 10 | 15207 | 12951 | 9778 | 7543 | 8334 | 6460 | 4307 | 2153 | 431 | 78 | 3.9 |
| Day 11 | 13534 | 11527 | 8702 | 6714 | 7417 | 5749 | 3833 | 1916 | 383 | 69 | 3.6 |
| Day 12 | 16918 | 14408 | 10878 | 8392 | 9272 | 7187 | 4791 | 2396 | 479 | 86 | 3.8 |
| Day 13 | 16072 | 13688 | 10334 | 7972 | 8808 | 6827 | 4551 | 2276 | 455 | 82 | 3.8 |
| Day 14 | 14304 | 12182 | 9197 | 7095 | 7839 | 6076 | 4051 | 2025 | 405 | 73 | 3.5 |
| Day 15 | 16879 | 14375 | 10853 | 8373 | 9250 | 7170 | 4780 | 2390 | 478 | 86 | 3.8 |
| Day 16 | 17047 | 14519 | 10962 | 8456 | 9343 | 7242 | 4828 | 2414 | 483 | 87 | 4.0 |
| Day 17 | 12445 | 10599 | 8002 | 6173 | 6820 | 5286 | 3524 | 1762 | 352 | 63 | 3.6 |
| Day 18 | 14933 | 12718 | 9602 | 7408 | 8184 | 6344 | 4229 | 2115 | 423 | 76 | 3.8 |
| Day 19 | 15829 | 13482 | 10178 | 7852 | 8675 | 6724 | 4483 | 2241 | 448 | 81 | 3.9 |
| Day20 | 17729 | 15099 | 11400 | 8795 | 9716 | 7531 | 5021 | 2510 | 502 | 90 | 4.0 |
| Day21 | 20211 | 17213 | 12996 | 10026 | 11077 | 8586 | 5724 | 2862 | 572 | 103 | 4.0 |
| Day22 | 21828 | 18590 | 14035 | 10828 | 11963 | 9272 | 6182 | 3091 | 618 | 111 | 4.1 |
| Day23 | 24884 | 21193 | 16000 | 12344 | 13638 | 10571 | 7047 | 3524 | 705 | 127 | 4.2 |
| Day24 | 22893 | 19497 | 14720 | 11356 | 12547 | 9725 | 6483 | 3242 | 648 | 117 | 4.3 |
| Day25 | 21977 | 18718 | 14132 | 10902 | 12045 | 9336 | 6224 | 3112 | 622 | 112 | 4.4 |

### Example 4: Polymerization

The acrylamide solution as prepared in examples 2 and 3 during day 3 are evaluated in a bulk polymerization. The acrylamide solutions 425 g is diluted with 560g water. The solutions are cooled down to 1°C. Under stirring 20 mg of sodium persulfate, 10mg of sodium hypophosphite and 1.2 g of 2,2'-Dimethyl-2,2'-azopropionitrile are added. The pH is adjusted with acetic acid solution to a value of 4. The solutions are loaded in a 2 L Dewar jar. A nitrogen degassing is operated with a deep nozzle in the solution for 30 min. 10 mg of Mohr salt solution at 50% is then added to the mixture. The polymerisation is generally initiated after 5 min with a continuous degassing. The temperature is naturally increased by 85°C. the polymer is let for 3h ageing. The inner polymer is then cut, crushed, and dried. A polymer powder is finally obtained. A UL viscosity measurement is done for example 2 and 3. The value are reported in table 6.

The UL viscosity is measured using a Brookfield viscometer equipped with a UL adapter, the unit of which rotates at 60 revolutions/minute (0.1 percent by weight of polymer in a 1M saline sodium chloride solution) between 23 and 25°C.

**Table 6: UL viscosity measured.**

| UL viscosity (cps) | Example 2 | Example 3 |
|---|---|---|
| Day 3 | 4.4 | 3.2 |

In case of example 2 the polymer exhibit a higher UL viscosity compared to example 3.

### Example 5: Stability test

The acrylamide solutions as prepared in examples 2 and 3 are evaluated in stability for self-polymerisation. For each production day 4 closed jars of 50 mL of the acrylamide solution with a 2x5 cm; 1 mm thick carbon steel plate, are placed in a 70°C oven. The appearance of the solutions is checked every day, when more than 50% of the solutions start to polymerise with an enhanced viscosity, the polymerization delay is recorded. The polymerization delays are reported (in days) in the table 7. In case of the acrylamide solution production as described in example 2 the average stability delay is improved.

**Table 7: Stability delay measured at 70°C for each production day.**

| | Stability (in days) | |
|---|---|---|
| Production day | Example 2 | Example 3 |
| Day 1 | 80 | 53 |
| Day2 | 71 | 49 |
| Day3 | 71 | 47 |
| Day4 | 80 | 49 |
| Day5 | 57 | 53 |
| Day6 | 71 | 49 |
| Day7 | 67 | 56 |
| Day8 | 69 | 51 |
| Day9 | 57 | 51 |
| Day 10 | 85 | 51 |
| Day 11 | 67 | 56 |
| Day 12 | 57 | 53 |
| Day 13 | 57 | 53 |
| Day 14 | 57 | 57 |
| Day 15 | 74 | 53 |
| Day 16 | 74 | 50 |
| Day 17 | 67 | 56 |
| Day 18 | 54 | 53 |
| Day 19 | 54 | 51 |
| Day20 | 54 | 50 |
| Day21 | 53 | 50 |
| Day22 | 63 | 49 |
| Day23 | 57 | 48 |
| Day24 | 74 | 47 |
| Day25 | 80 | 45 |

## Claims

1. A process for producing an aqueous acrylamide solution, comprising:
(a) combining water and at least one biocatalyst having nitrile hydratase activity to provide a slurry;
(b) feeding acrylonitrile into a reactor comprising said slurry to provide a reaction mixture; and
(c) monitoring said reaction mixture by online GC to measure a concentration of acrylonitrile in reactor's headspace with several detection technologies selected in the group consisting of Flame Ionization Detector, Mass Spectrometry, Thermal Conductivity Detector, Electron Capture Detector, Nitrogen-Phosphorus detector and vacuum ultraviolet detector.

2. The process of claim 1, wherein one or more of (i) the acrylonitrile feed rate, (ii) the amount of water, (iii) the at least one biocatalyst and/or the amount thereof or (iv) the temperature and/or the pH is adjusted during the reaction process based on the detected concentration of acrylonitrile in the reactor headspace.

3. The process of any one of the foregoing claims, wherein the sampling loop compressor is not a temperature-controlled loop connected thereto to GC online system.

4. The process of any one of the foregoing claims, wherein the concentration of acrylonitrile is within a range of 0 to 15000 ppmv and is measured by online GC from reactor's headspace with an accuracy of at least ± 100 ppmv, more specifically at least ± 50 ppmv;

5. The process of any one of the foregoing claims, wherein the concentration of acrylonitrile is within a range of 0 to 5000 ppmv and is measured by GC online in reactor's headspace with an accuracy of at least ± 50 ppmv, more specifically at least ± 10 ppmv.

6. The process of any one of the foregoing claims, wherein the concentration of acrylonitrile is within a range of 0 to 300 ppmv and is measured by GC online in reactor's headspace spectroscopy with an accuracy of at least ±10 ppmv.

7. The process of any one of the foregoing claims, wherein the on residual concentration of acrylonitrile as measured by GC in reactor's headspace is at most 1000 ppmv, at most 500 ppmv, at most 150 ppmv, or more specifically at most 50 ppmv.

8. The process of any one of the foregoing claims, wherein
- the acrylonitrile feed rate is adjusted during the process, thereby controlling acrylonitrile accumulation in the reactor headspace; and
- 38 % to 48 % of the total amount of acrylonitrile fed to the reactor is fed during the time period spanning 0 min to 60 min inclusive from the beginning of feeding acrylonitrile into the reactor.

9. The process of any one of the foregoing claims, wherein the reactor is a semi-batch reactor, a micro reactor, a continuous reactor, continuous reactors in series, or stirred tank reactors in series.

10. The process of any one of the foregoing claims, wherein:
- the biocatalyst comprises 0.0001 to 0.2 kg dry cells/m³ of the reaction mixture;
- the biocatalyst is a microbe selected from the group consisting of *Rhodococcus, Aspergillus, Acidovorax, Agrobacterium, Bacillus, Bradyrhizobium, Burkholderia, Escherichia, Geobacillus, Klebsiella, Mesorhizobium, Moraxella, Pantoea, Pseudomonas, Rhizobium, Rhodopseudomonas, Serratia, Amycolatopsis, Arthrobacter, Brevibacterium, Corynebacterium, Microbacterium, Micrococcus, Nocardia, Pseudonocardia, Trichoderma, Myrothecium, Aureobasidium, Candida, Cryptococcus, Debaryomyces, Geotrichum, Hanseniaspora, Kluyveromyces, Pichia, Rhodotorula, Comomonas,* and *Pyrococcus,* more preferably, the biocatalyst is selected from the group consisting of *Rhodococcus, Pseudomonas, Escherichia* and *Geobacillus* or a combination of at least two of any of the foregoing.

11. The process of any one of the foregoing claims, further comprising:
- measuring the temperature of the reaction mixture;
- maintaining the temperature of the reaction mixture within a range of 10 °C to 35 °C;further optionally such that the final concentration of acrylonitrile in the reactor headspace is at most 1000 ppmv.

12. An aqueous acrylamide solution wherein
- the color of the solution is equal or less than 20 Hazen measured with spectrophotometer PtCo (455 nm, cell path length: 10 00, 25°C) from 0.45 µm filtrated acrylamide sample;
- the concentration of acrylamide solution is from 35 to 55 weight %, more specifically from 38 to 40 weight %;
- the turbidity of the solution is equal or less than 15 NTU.

13. Use of the aqueous acrylamide solution of claim 12 for the manufacture of polyacrylamides, or N-methylenebisacrylamide or N-methylolacrylamide or N,N'-methyleneacrylamido-2-methylacrylamide.
